# EUROPÄISCHE PATENTSCHRIFT

(11) **EP 0 633 747 B1**
(45) Veröffentlichungstag und Bekanntmachung des Hinweises auf die Patenterteilung: **25.09.1996**
(21) Anmeldenummer: 93906609.8
(22) Anmeldetag: 25.03.1993
(51) Int. Cl.: A61B 17/12

(54) **ABSCHNÜRVORRICHTUNG FÜR KÖRPERTEILE**
CONSTRICTING DEVICE FOR PARTS OF THE BODY
DISPOSITIF DE CONSTRICTION POUR PARTIES DU CORPS

(30) Priorität: 28.03.1992 DE 4210255; 02.07.1992 DE 9208869 U
(43) Veröffentlichungstag der Anmeldung: 18.01.1995
(73) Patentinhaber: KIMETEC GMBH MEDIZINTECHNIK, D-71254 Ditzingen (DE)
(72) Erfinder: KIRCHNER, Claudia, D-7145 Markgröningen (DE)
(74) Vertreter: Jeck, Anton, Dipl.-Ing.
(86) Internationale Anmeldenummer: EP9300721
(87) Internationale Veröffentlichungsnummer: WO9319677

(56) Entgegenhaltungen:
- WO-A-88/00456
- DE-A- 3 602 778
- FR-A- 2 321 264

## Beschreibung

Die Erfindung betrifft eine Abschnürvorrichtung für Körperteile, mit einem Schloßgehäuse, das einen Bodenteil, zwei Seitenwände und an der Oberseite eine Abdeckwand aufweist, mit einem mit einem Ende am Schloßgehäuse mittels eines Rastschuhs angekoppelten Abschnürband, das unter Bildung einer Schlaufe mit seinem anderen freien Ende zwischen dem Bodenteil und der Abdeckwand durch das Schloßgehäuse geführt ist, und mit einer Wippe, die zum Festklemmen des Bandes im Schloßgehäuse schwenkbar gelagert ist und einen über dem Bodenteil angeordneten plattenartigen Abschnitt, über den das freie Ende des Bandes geführt und im festgeklemmten Zustand gegen ein darüber angeordnetes Widerlager des Schloßgehäuses gedrückt ist, und einen im Bereich der der Schlaufe abgewandten Stirnseite des plattenartigen Abschnitts angebrachten Betätigungsteil aufweist, der in seinem Ansatzbereich etwa über die Breite des plattenartigen Abschnitts reicht und eine Durchführöffnung für das freie Ende des Bandes besitzt, wobei die Wippe über seitliche Achsabschnitte in entsprechenden Löchern der Seitenwände und/oder auf einer auf dem Bodenteil vorgesehenen Leiste gelagert ist.

Eine Abschnürvorrichtung dieser Art ist in der DE 36 02 778 A1 als bekannt ausgewiesen. Bei dieser bekannten Vorrichtung ist über der Wippe lediglich die Oberseite des Schloßgehäuses vorgesehen, die als Widerlager zum Festklemmen des Abschnürbandes dient. Das Betätigungsteil zum Lockern der Schlaufe ragt nach vorne an dem Schloßgehäuse vor. Durch diese Ausbildung wird die Handlichkeit benachteiligt.

Eine weitere Abschnürvorrichtung ist in der DE 38 40 007 A1 als bekannt ausgewiesen. Das Abschnürband ist auf seiner einen Seite mit einem Rastschuh versehen, der in einem Schloßgehäuse rastend gekoppelt ist, und mit der anderen Seite unter Bildung einer Schlaufe über den Bodenteil des Schloßgehäuses geführt und mittels eines Klemmhebels gegen den Boden festgeklemmt.

Über die Ausbildung des Klemmhebels und dessen Betätigung sind keine näheren Ausführungen gemacht.

Eine der vorstehenden Abschnürvorrichtung verwandte Vorrichtung ist in der DE 38 39 794 A1 beschrieben, in der es hauptsächlich um die Aufnahmen in den Gehäuseseitenwänden für die Achsstifte des schwenkbar gelagerten Klemmhebels geht. Auch hier sind über den Klemmechanismus an sich keine näheren Ausführungen gemacht.

Auch bei weiteren Abschnürvorrichtungen, wie sie die EP 0 196 646 A2, die DE 34 31 728 A1, die DE 25 36 620 C2, die DE 25 41 433 C2, die DE 33 14 099 C2 sowie die DE 36 24 112 A1 zeigen, bestehen vorliegende Probleme, wobei die Abschnürvorrichtungen insbesondere aufgrund der Klemmvorrichtungen relativ aufwendig ausgeführt sind oder die Handhabbarkeit verbesserungsbedürftig ist.

Der Erfindung liegt die Aufgabe zugrunde, eine gattungsgemäße Abschnürvorrichtung derart weiterzubilden, daß eine handliche und leicht mit einer Hand betätigbare Abschnürvorrichtung bereitgestellt wird.

Diese Aufgabe wird mit den im Patentanspruch 1 angegebenen Merkmalen gelöst.

Gemäß dem Patentanspruch 1 ist also vorgesehen, daß als Widerlager über dem plattenartigen Abschnitt parallel zum Bodenteil fest verbunden mit dem Schloßgehäuse eine Mittelwand angeordnet ist, die von der Oberseite des plattenartigen Abschnittes bei dessen paralleler Stellung einen lichten Abstand aufweist, der größer ist als die Dicke des Bandes, daß die Abdeckwand des Schloßgehäuses auf ihrer von der Schlaufe abgewandten Seite gegenüber dem plattenartigen Abschnitt der Wippe verkürzt ist, und daß der Betätigungsteil nach hinten abgebogen ist und bis in die Nähe dieser Seite der Abdeckwand verläuft und gegenüber der Oberseite der Abdeckwand im Klemmzustand leicht vorsteht oder mit dieser bündig ist.

Hierdurch erhält die Abschnürvorrichtung nicht nur eine sehr kompakte, handliche Form, sondern es wird auch die Bedienbarkeit bequemer und genauer, da der Druck mit dem Daumen auf das Betätigungsteil im wesentlichen zentral auf das in der Hand ruhende Schloßgehäuse erfolgt. Hierdurch wird ein langsames Entstauen auf einfache Weise ermöglicht, ohne daß der Benutzer sich auf die Betätigung der Abschnürvorrichtung zu sehr konzentrieren muß und von seiner eigentlichen Aufgabe - der Kontrolle der Meßwerte und Beobachtung des Patienten - abgelenkt wird. Auch kommen die erfindungsgemäßen Maßnahmen dem optischen Gesamteindruck der Abschnürvorrichtung zugute.

Eine besonders einfache Ausbildung des Schloßgehäuses wird dadurch erreicht, daß der plattenartige Abschnitt, der Betätigungsteil und die seitlichen Achsabschnitte einstückig geformt sind.

Eine gute Hebelwirkung für das Festklemmen des Bandes und seine Freigabe mit dem Betätigungsteil ergibt sich, wenn vorgesehen ist, daß der plattenartige Abschnitt sich über die Länge des Bodenteils erstreckt oder mit seiner der Schlaufe zugewandten Stirnseite über diesen vorsteht.

Wenn eine Rastzunge des in das Schloßgehäuse eingeführten Rastschuhs in eine Aussparung der Abdeckwand eingerastet ist und mittels eines in der Aussparung angeordneten Druckteils ausrastbar ist, kann auch das Bandende mit dem Rastschuh leicht mit dem Daumen der das Schloßgehäuse haltenden Hand gelöst werden, ohne mit der Hand umgreifen zu müssen.

Die Maßnahme, daß die vordere Stirnseite des Rastschuhs federnd ausgebildet ist und gegen ein festes Widerlager drückt, oder daß das Widerlager federnd ausgebildet ist, ermöglicht ein leichtes Einrasten und Lösen des Rastschuhs durch Betätigen des Druckteils, da der Rastschuh leicht gegen die Federkraft weiter nach vorne gleiten kann. Ferner wird sichergestellt, daß sich die Rastzunge nach Einrasten infolge der von der Federkraft bewirkten Vorspannung stets ohne Spiel an der Rückwand der Aussparung abstützt. Beim Niederdrücken zum Lösen des Rastschuhs gleitet dieser infolge der Vorspannung zurück und rastet nicht ungewollt wieder ein.

Eine gute Führung für den Rastschuh wird dadurch erzielt, daß er mit seiner Unterseite auf der Oberseite der Mittelwand aufliegt.

Die handliche Form des Schloßgehäuses wird dadurch begünstigt, daß der Rastschuh einen Anschlagteil aufweist, der bei eingeführtem Rastschuh an der der Schlaufe zugekehrten Seite des Schloßgehäuses anliegt, wobei seine Oberkante vorzugsweise bündig ist mit der Oberseite des Schloßgehäuses.

Ein Austausch des Bandes ohne Rastschuh wird dadurch ermöglicht, daß der Anschlagteil vorzugsweise an seiner Unterseite eine hinterschnittene Nut aufweist, wobei die lichte Weite des hinterschnittenen Teils etwa der doppelten Stärke des Bandes entspricht, und daß das Band mit umgeschlagenem Ende in den hinterschnittenen Teil eingebracht ist, wobei das Bandende vorzugsweise mit einer steifen Bandklemme versehen ist, die ein Ausfransen verhindert und eine Keilwirkung bei Zugbeanspruchung erzeugt und darüber hinaus das Eindringen des Bandes in die hinterschnittene Nut erleichtert.

Die Maßnahme, daß an dem Schloßgehäuse an seiner der Schlaufe zugewandten Seite Winkelschrägen in der Weise ausgebildet sind, daß diese Seite zwischen den Einführöffnungen für den Rastschuh und für das Band eine in Richtung zur Schlaufe vortretende abgerundete Kante aufweist, ermöglicht einerseits ein leichtes Einführen des Rastschuhs in das Schloßgehäuse, da die Einführöffnung besser zugänglich ist, und andererseits wird ein Einziehen von Hautfalten in die Bandeinführöffnung vermieden, da sich die abgerundete Kante gegen den Körperteil abstützt.

Ist vorgesehen, daß die Rückseite des Schloßgehäuses einen Vorsprung aufweist, der bei eingeführtem Rastschuh in eine auf der zugekehrten Seite des Anschlagteils eingebrachte, mit einem federnden Element versehene Aussparung gedrückt ist, oder daß umgekehrt die Rückseite des Schloßgehäuses die Aussparung mit dem federnden Element und die zugekehrte Seite des Anschlagteils den Vorsprung aufweisen, und daß eine Rastzunge des in das Schloßgehäuse eingeführten Rastschuhs in eine Aussparung der Abdeckwand eingerastet ist und mittels eines in der Aussparung angeordneten Druckteils ausrastbar ist.

Durch das federnde Element und den dagegen gedrückten Vorsprung wird beim Lösen der Rastzunge mittels des Druckteils der gesamte Rastschuh nach hinten aus der Raststellung gedrückt, d.h. die Schlaufe wird durch lediglich kurze Betätigung des Druckteils zuverlässig geöffnet.

Vorteilhaft ist dabei die Ausbildung derart, daß das federnde Element gummielastisch ist und in der Aussparung liegt oder als Blattfeder ausgebildet ist.

Die Erfindung wird nachstehend anhand eines Ausführungsbeispiels unter Bezugnahme auf die Zeichnung näher erläutert. Es zeigen:
- Fig. 1: ein Ausführungsbeispiel der Abschnürvorrichtung in Längsschnitt und
- Fig. 2: eine Draufsicht der Abschnürvorrichtung nach Fig. 1, wobei der Rastschuh herausgezogen ist.

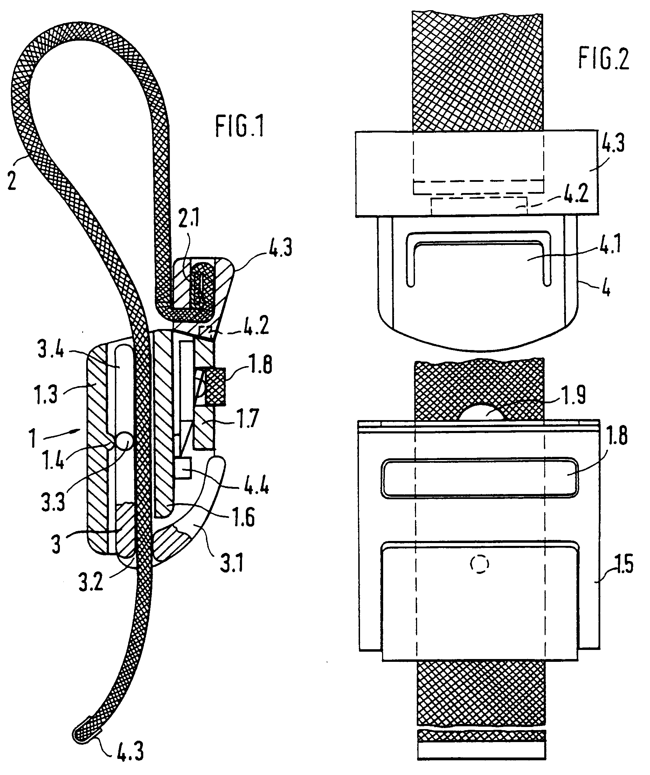

Das in Fig. 1 gezeigte Ausführungsbeispiel einer Abschnürvorrichtung zeigt als Hauptbestandteile ein Schloßgehäuse 1, ein zu einer Schlaufe umgelegtes und durch das Schloßgehäuse 1 geführtes Band 2, eine teilweise im Schloßgehäuse 1 angeordnete Wippe 3 sowie einen in das Schloßgehäuse 1 eingeführten Rastschuh 4, an dessen rückseitigem Ende das Band 2 angebracht ist.

Das Schloßgehäuse 1 besitzt einen Bodenteil 1.3, auf dem etwa in der Mitte eine über die Breite verlaufende abgerundete Leiste 1.4 angeordnet ist, eine an der Oberseite befindliche Abdeckwand 1.7, zwei Seitenwände 1.5 (vgl. Fig. 2) sowie an der hinteren, der Schlaufe zugewandten Stirnseite und der vorderen Stirnseite eine Öffnung zum Durchführen des Bandes 2. Das vordere Bandende kann mit einer steifen Klemme 4.3, z.B. aus Metall, versehen sein. Zwischen dem Bodenteil 1.3 und der Abdeckwand 1.7 ist eine Mittelwand 1.6 angeordnet, deren Unterseite der Oberseite des Bandes 2 zugekehrt ist und die zum Führen des Bandes 2 beiträgt und beim Einklemmen des Bandes 2 ein Widerlager bildet.

Im vorliegenden Ausführungsbeispiel sind Bodenteil 1.3, Mittelwand 1.6 und Abdeckwand 1.7 parallel zueinander ausgerichtet. Dies ist jedoch für die Funktion der Abschnürvorrichtung keine notwendige Voraussetzung. Beispielsweise kann, um eine festere oder losere Einklemmwirkung des Bandes 2 zu erzielen, die Mittelwand 1.6 schräg bezüglich des Bodenteils 1.3 ausgerichtet sein.

Auch das Vorhandensein der Leiste 1.4 und deren Anordnung in der Mitte des Bodenteils sind keine unbedingt notwendigen Voraussetzungen, wie unten ausgeführt wird.

Die der Schlaufe abgewandte Stirnseite des Bodenteils 1.3 steht bei dem in Fig. 1 gezeigten Ausführungsbeispiel weiter vor als diejenige der Mittelwand und diese wiederum ragt weiter vor als die Abdeckwand 1.7 an der Oberseite des Schloßgehäuses 1.

Die Wippe 3 besitzt einen zwischen dem Bodenteil 1.3 und der Mittelwand 1.6 eingesetzten plattenartigen Abschnitt 3.4, über den das Band 2 geführt ist. Die lichte Weite zwischen dem in seiner mittleren Schwenkstellung befindlichen plattenförmigen Abschnitt 3.4 und der Unterseite der Mittelwand 1.7 ist so groß, daß ein leichtes Durchführen des Bandes 2 sichergestellt ist und eine gute Klemmwirkung erzielt wird, wenn die Wippe 3 geschwenkt wird, d.h. sie ist vorzugsweise geringfügig größer als die Dicke des Bandes 2. Die Klemmwirkung entsteht, wenn das Band 2 an dem schlaufenseitigen Ende des plattenartigen Abschnittes 3.4 nach unten gezogen wird. Zur Verstärkung der Klemmwirkung kann der Bodenteil 1.3 schlaufenseitig kürzer sein als der plattenförmige Abschnitt 3.4. Um ein Rutschen im Klemmzustand zu vermeiden, kann die Oberseite des plattenartigen Abschnittes 3.4 und/oder die Unterseite der Mittelwand 1.6 aufgerauht oder geriffelt sein.

Im Bereich der der Schlaufe abgewandten vorderen Stirnseite des plattenartigen Abschnittes 3.4 ist ein nach oben geführtes und nach hinten abgebogenes Betätigungsteil 3.1 angeordnet, das sich im Ansatzbereich etwa über die Breite der Wippe 3 erstreckt und vorzugsweise einstückig an dem plattenartigen Abschnitt 3.4 zweckmäßigerweise über eine Rundung angeformt ist. Infolge des unterschiedlich weit vorragenden Bodenteils 1.3, der Mittelwand 1.6 und der Abdeckwand 1.7 kann das Betätigungsteil 3.1 von der vorderen Stirnseite des plattenartigen Abschnittes 3.4 in stetiger Krümmung nach hinten verlaufen. Das Ende des Betätigungsteils 3.1 grenzt nahezu an die vordere Stirnseite der Abdeckwand 1.7 und steht über deren Oberseite vor oder ist mit dieser bündig, so daß es zum Lösen des festgeklemmten Bandes leicht niedergedrückt werden kann.

Auf jeder Längsseite des plattenartigen Abschnittes 3.4 der Wippe 3 steht ein Achsabschnitt 3.3 vor, der in eine entsprechende Ausnehmung jeder Seitenwand 1.5 des Schloßgehäuses 1 eingesetzt ist. Vorzugsweise sind die Achsabschnitte 3.3 in Form von Zapfen einstückig an dem plattenartigen Abschnitt 3.4 angeformt.

Wenn die Leiste 1.4 auf dem Bodenteil 1.3 des Schloßgehäuses 1 vorgesehen ist, reicht es, daß die Ausnehmungen der Seitenwände für die Zapfen länglich ausgebildet sind und ein Verschieben der Wippe 3 nach oben, in Quer- und in Längsrichtung verhindern. Für die Schwenkbewegung der Wippe 3 und ihre Abstützung nach unten dient dann allein die Leiste 1.4. Ist die Leiste bzw. eine entsprechende Abstützung nicht vorgesehen, so müssen die Ausnehmungen in den Seitenwänden als entsprechende Bohrungen ausgebildet sein, und zwar als Sackloch- oder Durchgangsbohrungen.

Sind die Seitenwände am Schloßgehäuse 1 angeformt, so kann die Wippe 3 bei Wahl nachgiebigen Materials, wie z.B. Kunststoff, in das Schloßgehäuse 1 eingedrückt und mit den Achsabschnitten 3.3 in die Ausnehmungen der Seitenwände 1.5 eingerastet bzw. eingeschnappt werden. Zum Lösen kann ein Werkzeug vorgesehen werden, das durch Öffnungen in dem Bodenteil 1.3 an den Innenseiten der Seitenwände 1.5 vorbeigeführt wird und an den Achsabschnitten 3.3 angreift.

Eine andere Möglichkeit ist, die Seitenwände lösbar auszubilden, so daß sie bei in das Schloßgehäuse 1 eingeschobener Wippe 3 aufgesetzt werden können.

Wie aus Fig. 2 ersichtlich, weist der Rastschuh 4 eine Rastzunge 4.1 und einen das Band 2 aufnehmenden Anschlagteil 4.3 auf, der im eingeschobenen Zustand des Rastschuhs 4 an der der Schlaufe zugekehrten Rückseite des Schloßgehäuses 1 anliegt (s. Fig. 1). Außerdem kann der Rastschuh 4 eine federnd ausgebildete vordere Stirnseite aufweisen.

Die Rückseite des Schloßgehäuses 1 weist vorteilhaft, insbesondere wenn die vordere Stirnseite des Rastschuhs 4 nicht federnd ausgebildet ist, einen Vorsprung 1.9 auf, der angeformt sein kann. Im vollständig eingeführten Zustand des Rastschuhs 4 drückt dieser gegen ein federndes Element 4.2, das in einer Aussparung in der der Rückseite des Schloßgehäuses 1 zugekehrten Seite des Anschlagteils 4.3 eingebracht ist. Vorliegend füllt das federnde Element 4.2 die Aussparung aus und ist gummielastisch ausgebildet. Aber auch eine Blattfeder oder ein ähnliches Element, das in oder an der Aussparung vorgesehen sein kann, vermag eine vergleichbare Funktion zu erfüllen. Umgekehrt kann natürlich das federnde Element an der Rückseite des Gehäuses angeordnet und das Gegenelement am Anschlagteil vorgesehen sein. Wichtig ist, daß der Rastschuh 4 durch die Wirkung des federnden Elementes 4.2 und das dagegengedrückte Gegenelement 1.9 beim Eindrücken der Rastzunge 4.1 zuverlässig zurückgeschoben wird, um ein erneutes, nicht beabsichtigtes Einrasten der Rastzunge zu vermeiden.

Die Rastzunge 4.1 rastet beim Einschieben des Rastschuhs 4 in das Schloßgehäuse 1 in eine Aussparung ein, die in die Abdeckwand 1.7. eingearbeitet ist, und stützt sich mit ihrem hinteren Teil an einer Wand der Aussparung gegen eine Zugbelastung des Bandes 2 ab. Die federnde Rastzunge 4.1 ist mittels eines in der Aussparung angeordneten Druckteils 1.8, das über die Oberseite der Abdeckwand 1.7 ragt oder mit dieser bündig ist, eindrückbar, so daß der Rastschuh 4 ausgerastet wird. Um auch unter Zugbelastung ein leichtes Ausrasten zu ermöglichen, ist auf der Oberseite der Rastzunge 4.1 ein abgerundeter Abschnitt vorgesehen, der ein leichtes Gleiten beim Niederdrücken des Druckteils 1.8 gewährleistet, und ferner kann sich, wenn die Stirnseite 4.2 des Rastschuhs 2 federnd ausgebildet ist, dieser leicht gegen den Anschlag nach vorne bewegen, während die hintere Seite der Rastzunge 4.1 an der Abstützwand vorbeigleitet und ein leichtes Vorschieben des Rastschuhs bewirkt. Selbstverständlich darf hierbei der Anschlagteil nicht fest am Schloßgehäuse anliegen. Sobald die Rastzunge 4.1 vollständig niedergedrückt ist, kann der Rastschuh unterstützt durch die Federwirkung zurückgleiten und es wird dabei ein unbeabsichtigtes Wiedereinrasten verhindert.

Der Rastschuh 4 liegt mit seiner Unterseite im eingeschobenen Zustand auf der Oberseite der Mittelwand 1.6 auf. Zum Führen des Rastschuhs 4 können alternativ auch lediglich seitliche Führungen vorgesehen sein. Die Oberseite des Anschlagteils 4.3 fluchtet mit der Oberseite der Abdeckwand 1.7.

Die der Schlaufe zugewandte Seite des Schloßgehäuses 1 ist nach oben und unten abgeschrägt, wobei die Kante dieser Winkelschrägen zwischen den Einführöffnungen für den Rastschuh und für das freie Ende des Bandes 2 liegt und nach außen zur Schlaufe gerichtet ist. Hierdurch ergibt sich einerseits eine gute Führung für den Rastschuh beim Einführen und andererseits wird ein Einziehen von Hautfalten praktisch ausgeschlossen, da sich die abgerundete Kante an dem Körperteil abstützen kann.

Der Anschlagteil 4.3 besitzt vorzugsweise an seiner Unterseite eine Nut, die hinterschnitten ist, um das Band 2 darin festlegen zu können. Das Band 2 ist mit einem umgeschlagenen Ende in den hinterschnittenen Teil eingelegt, wozu dessen lichte Weite etwa der doppelten Stärke des Bandes 2 entspricht. Wird ein Zug auf das Band 2 ausgeübt, so versucht es sich aufzuwölben und wird dadurch fest eingeklemmt, insbesondere wenn am Bandende eine steife Klemme vorhanden ist. Andererseits kann das Band 2 im gelockerten Zustand leicht von dem Anschlagteil 4.3 gelöst werden, so daß ein Auswechseln des Bandes 2 ohne Verlust des Rastschuhs 4 ermöglicht ist.

Insgesamt besitzt die Abschnürvorrichtung eine handliche, leicht betätigbare Ausgestaltung. Sie ist infolge der kompakten Bauweise leicht in einer Hand zu halten, wobei sowohl die eingerastete Seite des Bandes 2 als auch sein eingeklemmter Teil mit dem Daumen dieser Hand leicht freigebbar ist.

## Patentansprüche

1. Abschnürvorrichtung für Körperteile, mit einem Schloßgehäuse, das einen Bodenteil, zwei Seitenwände und an der Oberseite eine Abdeckwand aufweist, mit einem mit einem Ende am Schloßgehäuse mittels eines Rastschuhs angekoppelten Abschnürband, das unter Bildung einer Schlaufe mit seinem anderen freien Ende zwischen dem Bodenteil und der Abdeckwand durch das Schloßgehäuse geführt ist, und mit einer Wippe, die zum Festklemmen des Bandes im Schloßgehäuse schwenkbar gelagert ist und einen über dem Bodenteil angeordneten plattenartigen Abschnitt, über den das freie Ende des Bandes geführt und im festgeklemmten Zustand gegen ein darüber angeordnetes Widerlager des Schloßgehäuses gedrückt ist, und einen im Bereich der der Schlaufe abgewandten Stirnseite des plattenartigen Abschnitts angebrachten Betätigungsteil aufweist, der in seinem Ansatzbereich etwa über die Breite des plattenartigen Abschnitts reicht und eine Durchführöffnung für das freie Ende des Bandes besitzt, wobei die Wippe über seitliche Achsabschnitte in entsprechenden Löchern der Seitenwände und/oder auf einer auf dem Bodenteil vorgesehenen Leiste gelagert ist,
dadurch gekennzeichnet,
daß als Widerlager (1.6) über dem plattenartigen Abschnitt (3.4) parallel zum Bodenteil (1.3) fest verbunden mit dem Schloßgehäuse (1) eine Mittelwand (1.6) angeordnet ist, die von der Oberseite des plattenartigen Abschnittes (3.4) bei dessen paralleler Stellung einen lichten Abstand aufweist, der größer als die Dicke des Bandes (2) ist, daß die Abdeckwand (1.7) des Schloßgehäuses (1) auf ihrer von der Schlaufe abgewandten Seite gegenüber dem plattenartigen Abschnitt (3.4) der Wippe (3) verkürzt ist, und
daß der Betätigungsteil (3.1) nach hinten abgebogen ist und bis in die Nähe dieser Seite der Abdeckwand (1.7) verläuft und gegenüber der Oberseite der Abdeckwand (1.7) im Klemmzustand leicht vorsteht oder mit dieser bündig ist.

2. Abschnürvorrichtung nach Anspruch 1,
dadurch gekennzeichnet,
daß der plattenartige Abschnitt (3.4), der Betätigungsteil (3.1) und die seitlichen Achsabschnitte (3.3) einstückig geformt sind.

3. Abschnürvorrichtung nach Anspruch 1 oder 2,
dadurch gekennzeichnet,
daß der plattenartige Abschnitt (3.4) sich über die Länge des Bodenteils (1.3) erstreckt oder mit seiner der Schlaufe zugewandten Stirnseite über diesen vorsteht.

4. Abschnürvorrichtung nach einem der vorhergehenden Ansprüche,
dadurch gekennzeichnet,
daß eine Rastzunge (4.1) des in das Schloßgehäuse (1) eingeführten Rastschuhs (4) in eine Aussparung der Abdeckwand (1.7) eingerastet ist und mittels eines in der Aussparung angeordneten Druckteils (1.8) ausrastbar ist.

5. Abschnürvorrichtung nach Anspruch 4,
dadurch gekennzeichnet,
daß die vordere Stirnseite (4.2) des Rastschuhs (4) federnd ausgebildet ist und gegen ein festes Widerlager (4.4) drückt, oder
daß das Widerlager (4.4) federnd ausgebildet ist.

6. Abschnürvorrichtung nach Anspruch 4 oder 5,
dadurch gekennzeichnet,
daß das Druckteil (1.8) gegenüber der Oberseite der Abdeckwand (1.7) des Schloßgehäuses (1) leicht vorsteht oder mit dieser bündig ist.

7. Abschnürvorrichtung nach einem der vorhergehenden Ansprüche,
dadurch gekennzeichnet,
daß der Rastschuh (4) mit seiner Unterseite auf der Oberseite der Mittelwand (1.6) aufliegt.

8. Abschnürvorrichtung nach einem der vorhergehenden Ansprüche,
dadurch gekennzeichnet,
daß der Rastschuh (4) einen Anschlagteil (4.3) aufweist, der bei eingeführtem Rastschuh an der der Schlaufe zugekehrten Seite des Schloßgehäuses (1) anliegt, wobei seine Oberkante vorzugsweise bündig ist mit der Oberseite (1.7) des Schloßgehäuses (1).

9. Abschnürvorrichtung nach Anspruch 8,
dadurch gekennzeichnet,
daß der Anschlagteil (4.3) vorzugsweise an seiner Unterseite eine hinterschnittene Nut aufweist, wobei die lichte Weite des hinterschnittenen Teils etwa der doppelten Stärke des Bandes (2) entspricht, und
daß das Band (2) mit umgeschlagenem Ende in den hinterschnittenen Teil eingebracht ist.

10. Abschnürvorrichtung nach Anspruch 9,
dadurch gekennzeichnet,
daß das umgeschlagene Ende des Bandes (2) mit einer Klemme (2.1) versehen ist.

11. Abschnürvorrichtung nach einem der Ansprüche 1 bis 10,
dadurch gekennzeichnet,
daß an dem Schloßgehäuse (1) an seiner der Schlaufe zugewandten Seite Winkelschrägen in der Weise ausgebildet sind, daß diese Seite zwischen den Einführöffnungen für den Rastschuh (4) und für das Band (2) eine in Richtung zur Schlaufe vortretende abgerundete Kante aufweist.

12. Abschnürvorrichtung nach einem der Ansprüche 1 bis 11,
dadurch gekennzeichnet,
daß die Rückseite des Schloßgehäuses (1) einen Vorsprung (1.9) aufweist, der bei eingeführtem Rastschuh (4) in eine auf der zugekehrten Seite des Anschlagteils (4.3) eingebrachte, mit einem federnden Element (4.2) versehene Aussparung gedrückt ist, oder daß umgekehrt die Rückseite des Schloßgehäuses die Aussparung mit dem federnden Element und die zugekehrte Seite des Anschlagteils den Vorsprung aufweisen, und
daß eine Rastzunge (4.1) des in das Schloßgehäuse (1) eingeführten Rastschuhs (4) in eine Aussparung der Abdeckwand (1.7) eingerastet ist und mittels eines in der Aussparung angeordneten Druckteils (1.8) ausrastbar ist.

13. Abschnürvorrichtung nach Anspruch 12,
dadurch gekennzeichnet,
daß das federnde Element (4.2) gummielastisch ist und in der Aussparung liegt oder als Blattfeder ausgebildet ist.

## Claims

1. Constricting device for parts of the body, having a lock housing which includes a base member, two lateral walls and, on the upper side, a cover wall, having a constricting band, which is connected at one end to the lock housing by means of a locking shoe and is guided through the lock housing so as to form a loop with its other free end between the base member and the cover wall, and having a rocker, which is pivotably mounted in the lock housing for secure clamping of the band and includes a plate-like portion, which is disposed above the base member and over which the free end of the band is guided, said plate-like portion being urged towards an abutment of the lock housing when the band is in its securely clamped state, which abutment is disposed above said band, and includes an actuating member, which is mounted in the region of the end face of the plate-like portion remote from the loop and extends within its extension range substantially over the width of the plate-like portion, said actuating member having a through-aperture for the free end of the band, the rocker being mounted over lateral axial portions in corresponding holes in the lateral walls and/or on a strip provided on the base member, characterised in that a central wall (1.6) is disposed as an abutment (1.6) above the plate-like portion (3.4) parallel to the base member (1.3) so as to be securely connected to the lock housing (1), which central wall has an unobstructed spacing from the upper side of the plate-like portion (3.4) when the latter is in its parallel position, said spacing being greater than the thickness of the band (2), in that the cover wall (1.7) of the lock housing (1) is shorter on its side remote from the loop relative to the plate-like portion (3.4) of the rocker (3), and in that the actuating member (3.1) is bent rearwardly and extends to the vicinity of this side of the cover wall (1.7), said actuating member protruding slightly beyond the upper side of the cover wall (1.7) in the clamped state or being flush therewith.

2. Constricting device according to claim 1, characterised in that the plate-like portion (3.4), the actuating member (3.1) and the lateral axial portions (3.3) are integrally formed.

3. Constricting device according to claim 1 or 2, characterised in that the plate-like portion (3.4) extends over the length of the base member (1.3) or protrudes beyond said base member with its end face facing the loop.

4. Constricting device according to one of the preceding claims, characterised in that a locking tongue (4.1) of the locking shoe (4), which is inserted into the lock housing (1), is locked in position in a recess in the cover wall (1.7) and is releasable by means of a pressure member (1.8) disposed in the recess.

5. Constricting device according to claim 4, characterised in that the front end face (4.2) of the locking shoe (4) is resilient and presses against a fixed abutment (4.4), or in that the abutment (4.4) is resilient.

6. Constricting device according to claim 4 or 5, characterised in that the pressure member (1.8) protrudes slightly beyond the upper side of the cover wall (1.7) of the lock housing (1) or is flush with said upper side.

7. Constricting device according to one of the preceding claims, characterised in that the locking shoe (4) lies with its underside on the upper side of the central wall (1.6).

8. Constricting device according to one of the preceding claims, characterised in that the locking shoe (4) has a stop member (4.3), which abuts against the side of the lock housing (1) facing the loop when the locking shoe has been inserted, the upper edge of said locking shoe preferably being flush with the upper side (1.7) of the lock housing (1).

9. Constricting device according to claim 8, characterised in that the stop member (4.3) preferably has a recessed groove on its underside, the inside width of the recessed part corresponding to substantially twice the thickness of the band (2), and in that the band (2) is introduced into the recessed part by its folded-over end.

10. Constricting device according to claim 9, characterised in that the folded-over end of the band (2) is provided with a clip (2.1).

11. Constricting device according to one of claims 1 to 10, characterised in that angular inclinations are provided on the side of the lock housing (1) facing the loop in such a manner that this side has a rounded edge, protruding in a direction towards the loop, between the insert apertures for the locking shoe (4) and for the band (2).

12. Constricting device according to one of claims 1 to 11, characterised in that the rear side of the lock housing (1) has a projection member (1.9), which is urged into a recess provided in the facing side of the stop member (4.3) and provided with a resilient element (4.2) when the locking shoe (4) has been inserted, or in that, conversely, the rear side of the lock housing has the recess with the resilient element and the facing side of the stop member has the projection member, and in that a locking tongue (4.1) of the locking shoe (4), which is inserted into the lock housing (1), is locked in position in a recess in the cover wall (1.7) and is releasable by means of a pressure member (1.8) disposed in the recess.

13. Constricting device according to claim 12, characterised in that the resilient element (4.2) is rubber-elastic and lies in the recess or is configured as a leaf spring.

## Revendications

1. Dispositif de constriction pour parties du corps, avec un boîtier de fermeture qui présente un élément de base, deux parois latérales et une paroi de recouvrement à la face supérieure, avec un ruban de constriction accouplé par une extrémité par l'intermédiaire d'un patin d'encliquetage au boîtier de boucle, ruban qui avec la formation d'une boucle est par son autre extrémité, laquelle est libre, guidé au travers le boîtier de fermeture entre l'élément de base et la paroi de recouvrement, dispositif comportant une bascule montée à pivotement aux fins du serrage du ruban dans le boîtier de fermeture, ainsi qu'un tronçon en forme de plaque disposé au-dessus de l'élément de base, tronçon au-dessus est guidée l'extrémité libre du ruban et qui à l'état de serra est poussé sous pression contre une butée disposée dans le haut du boîtier de serrage, dispositif présentant un élément de commande situé dans la région de la face frontale du tronçon en forme de plaque qui n'est pas orientée vers la boucle, élément de commande qui dans sa région de base s'étend sensiblement sur toute la largeur du tronçon en forme de plaque et qui présente une ouverture de passage pour l'extrémité libre du ruban, la bascule étant par l'intermédiaire de tronçons axiaux latéraux dans des trous correspondants des parois latérales et/ou est monté sur une tringle prévue sur l'élément de base,
caractérisé
en ce que en tant que butée (1.6) est utilisée une paroi médiane (1.6) disposée au-dessus du tronçon en forme de plaque (3.4) parallèlement à l'élément de base (1.3) et fixée rigidement au boîtier de fermeture (1), paroi médiane (1.6) qui est distante de la face supérieure du tronçon en forme de plaque (3.4), lorsque celui-ci occupe une position parallèle, d'une longueur qui est supérieure à l'épaisseur du ruban (2),
en ce que la paroi de recouvrement (1.7) du boîtier de fermeture (1) est, sur son côté éloigné de la boucle, raccourcie par rapport au tronçon en forme de plaque (3.4) de la bascule (3), et
en ce que l'élément de commande (3.1) est incurvé vers l'arrière et s'étend jusqu'à près de ce côté de la paroi de recouvrement (1.7) et en ce que dans la position de serrage, l'élément de commande est situé légèrement en protubérance sur la face supérieure de la paroi de recouvrement (1.7) ou est à fleur avec celle-ci.

2. Dispositif de constriction suivant la revendication 1,
caractérisé
en ce que le tronçon en forme de plaque (3.4), l'élément de commande (3.1) et les tronçons axiaux latéraux (3.3.) sont formés en une pièce.

3. Dispositif de constriction suivant les revendications 1 ou 2,
caractérisé
en ce que le tronçon en forme de plaque (3.4) s'étend sur toute la longueur de l'élément de base (1.3) ou fait protubérance sur celui-ci par sa face frontale orientée vers la boucle.

4. Dispositif de constriction suivant l'une quelconque des revendications précédentes,
caractérisé
en ce qu'une languette d'encliquetage (4.1) du patin d'encliquetage (4) introduit dans le boîtier de fermeture (1) est encliquetée dans un évidement de la paroi de recouvrement (1.7) et peut être décliquetée au moyen d'un élément de pression (1.8) disposé dans cet évidement.

5. Dispositif de constriction suivant la revendication 1,
caractérisé
en ce que la face frontale antérieure (4.2) du patin d'encliquetage (4) est élastique et d'appuie sur une butée fixe (4.4), ou en ce que la butée (4.4) est élastique.

6. Dispositif de constriction suivant la revendication 6,
caractérisé
en ce que l'élément de pression (1.8) fait légèrement protubérance sur la face supérieure de la paroi de recouvrement (1.7) du boîtier de fermeture (1) ou est à fleur de celle-ci.

7. Dispositif de constriction suivant l'une quelconque des revendications précédentes,
caractérisé
en ce que le patin d'encliquetage (4) s'applique par sa face inférieure sur la face supérieure de la paroi médiane (1.6).

8. Dispositif de constriction suivant l'une quelconque des revendications précédentes,
caractérisé
en ce que le patin d'encliquetage (4) présente un élément de butée (4.3) lequel, lorsque le patin d'encliquetage est introduit, s'applique contre la face du boîtier de fermeture (1) orientée vers la boucle, l'arête supérieure de l'élément de butée étant de préférence à fleur de la face supérieure (1.7) du boîtier de fermeture (1).

9. Dispositif de constriction suivant la revendication 8,
caractérisé
en ce que l'élément de butée (4.3) présente de préférence à sa face inférieure une rainure en dépouille, la largeur intérieure de la partie en dépouille correspondant sensiblement au double de l'épaisseur du ruban (2), et en ce que le ruban (2) est par une extrémité rabattue introduit dans la partie en dépouille.

10. Dispositif de constriction suivant la revendication 9,
caractérisé
en ce que l'extrémité rabattue du ruban (2) est équipée d'une pince (2.1).

11. Dispositif de constriction suivant l'une quelconque des revendications de 1 à 10,
caractérisé
en ce que la face du boitier de fermeture (1) orientée vers la boucle présente des biseaux, de manière telle qu'entre les ouvertures d'admission pour le patin d'encliquetage (4) et pour le ruban (2), cette face présente une arête arrondie faisant protubérance en direction de la boucle.

12. Dispositif de constriction suivant l'une quelconque des revendications de 1 à 11,
caractérisé
en ce que la face postérieure du boîtier de fermeture (1) présente une protubérance (1.9) laquelle, lorsque le patin d'encliquetage (4) est introduit est appliqué sous pression sur un évidement doté d'un élément élastique (4.2), évidement pratiqué dans le côté opposé de l'élément de butée (4.3), ou inversement en ce que la face postérieure du boîtier de fermeture présente l'évidement avec l'élément élastique, tandis que la face opposée de l'élément de butée présente la protubérance, et
en ce qu'une languette d'encliquetage (4.1) du patin d'encliquetage (4) introduit dans le boîtier de fermeture (1) est encliqueté dans un évidement de la paroi de recouvrement (1.7) et peut être décliquetée au moyen, d'un élément de pression (1.8) disposé dans l'évidement.

13. Dispositif de constriction suivant la revendication 12,
caractérisé
en ce que l'élément élastique est à base de caoutchouc et est situé dans l'évidement ou bien a la forme d'une lame de ressort.
